# EUROPEAN PATENT APPLICATION

(11) **EP 4 567 620 A1**
(43) Date of publication of application: **11.06.2025**
(21) Application number: 23214384.2
(22) Date of filing: 05.12.2023
(51) Int. Cl.: G06F 16/21, G06F 16/215, G06N 3/091, G16H 10/60

(54) **METHODS AND SYSTEMS FOR AUTOMATED DATA-INTEGRATION**

(71) Applicant: Koninklijke Philips N.V., 5656 AG Eindhoven (NL)
(72) Inventor: STAPLETON, Shawn Arie Peter, Eindhoven (NL)
(74) Representative: Philips Intellectual Property & Standards

(57) **Abstract**

A method (300) for mapping data between a source data schema and a target data schema, comprising: extracting (320) and normalizing data to generate a normalized data set (462); analyzing (330), using a first trained machine learning algorithm (463), the normalized data set to identify a source data schema; deriving (340), using a second trained machine learning algorithm (464), mapping rules configured to transform data from the identified source data schema to the target schema; transforming (350), using the derived mapping rules, at least some data of the identified source data schema to data of the target schema; analyzing (360), using a schema validation algorithm (466), the transformed data to identify any mapping anomalies; and providing (370), to a user via a user interface, one or more of the identified source data schema, at least some of the data transformed to the target schema, and any identified mapping anomalies.

## Description

### FIELD OF THE INVENTION

The present disclosure is directed generally to methods and systems for automated mapping of data between a source data schema and a target data schema.

### BACKGROUND OF THE INVENTION

The exchange of patient health data among healthcare IT (HIT) applications, within and across healthcare organizations, is crucial to delivering high-quality, cost-efficient, and clinically impactful healthcare. To support data sharing, international data-sharing standards such as HL7/FHIR, IHE, and DICOM have been developed. However, the implementation of these standards is inconsistent as each HIT application has its own unique requirements to meet specific workflows and business logic. Additionally, both healthcare IT and medical system vendors use non-standard/custom data models to enable ad-hoc data exchange, further complicating the integration process. Consequently, healthcare IT and medical system vendors must support a complex and bespoke model of data integration across multiple hardware/software systems.

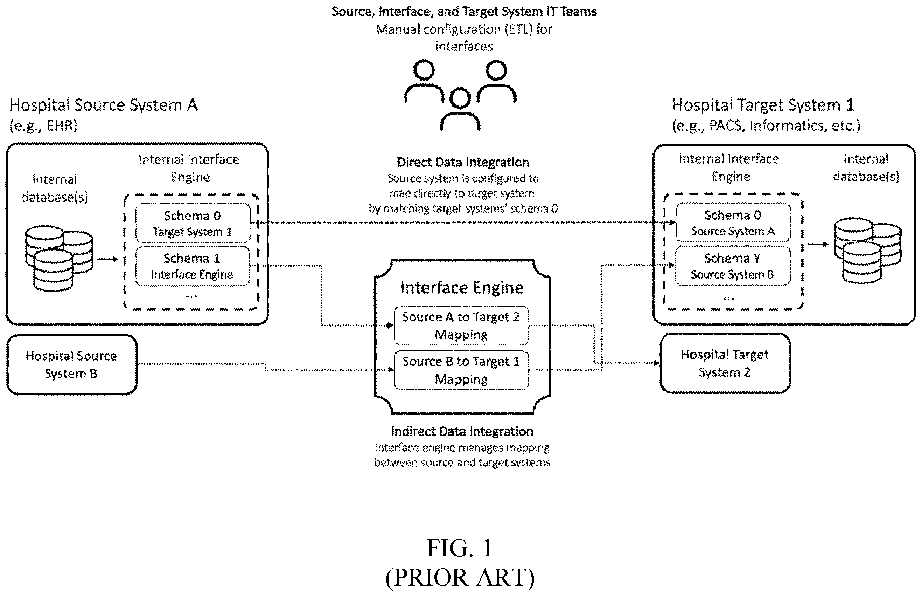

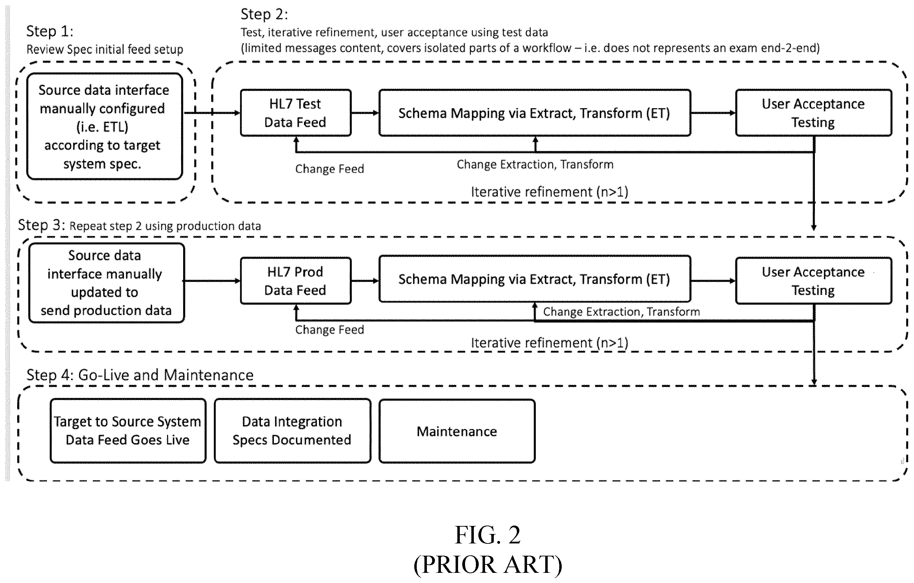

Figs. 1 and 2 is an illustrative example of the current manual process for data integration is inefficient and lacks automated tools to expedite the process. These traditional integration methods comprise four steps: (1) data schema and IT specification sharing and review; (2) schema mapping; (3) schema validation; and (4) acceptance, documentation, and go-live. This is a multi-step and iterative process that requires significant effort from experts of the source and the receiving systems. For example, steps 2 and 3 are performed iteratively using test data (i.e., sample data that does not contain PHI) and production data (real clinical data).

This manual integration process is repeated for each data integration between medical systems, necessitating substantial manual effort by both teams to facilitate data sharing across applications. Each data integration is unique, with significant variations in the integration requirements between different hospital sites being commonplace. Moreover, both standard and non-standard schema are not well documented, leading to inaccurate data schema specifications, further hindering efficient data integration. Typically, completing a single data integration between individual medical systems requires at least 10s to 100s hours of manual effort over at least 1 to 4 months. As a result, extensive training of IT staff over several months and deep experience from performing numerous data integrations is necessary for humans to accomplish data integrations efficiently.

### SUMMARY OF THE INVENTION

The diversity of HIT applications and the heterogeneity of hospital information systems make integrating data from across systems for application or IT installations a slow process vulnerable to errors and application or IT failures as a consequence of those errors. Thus, there is a continued need for methods and systems to automate the data integration process, including for the improved exchange of patient health data among HIT applications. The described embodiments solve this problem by increasing the speed, reliability, and consistency of installations, thereby reducing errors and increasing the speed with which providers can treat patients using the installed applications and/or IT offerings. Various embodiments and implementations described or otherwise envisioned herein are directed to a method and system for mapping data between a source data schema and a target data schema. A data mapping system extracts and normalizes data from one or more data sources within a source data schema to generate a normalized data set. A trained machine learning algorithm of the data mapping system analyzes the extracted data to identify a source data schema. A trained machine learning algorithm of the data mapping system then derives mapping rules configured to transform data from the identified source data schema to the target schema. The data mapping system transforms at least some data of the identified source data schema to data of the target schema using the derived mapping rules. A schema validation algorithm of the data mapping system analyzes the data transformed to the target schema to identify any mapping anomalies. The data mapping system then provides the identified source data schema, at least some of the data transformed to the target schema, and/or any identified mapping anomalies to a user via a user interface. The feedback is gathered through a standalone user-interface that prompts the user to provide their feedback, which can also be integrated into the schema validation/mapping workflow when validating ETL scripts.

According to an aspect of the inventive concept, a method for mapping data between a source data schema and a target data schema is provided. The method includes: (i) extracting and normalizing data from one or more data sources to generate a normalized data set, wherein the one or more data sources comprises the source data schema; (ii) analyzing, using a first trained machine learning algorithm, the normalized data set to identify the source data schema, wherein identifying the source data schema comprises identifying a general schema level, a data record level, and a data element level for the source data schema; (iii) deriving, using a second trained machine learning algorithm, mapping rules configured to transform data from the identified source data schema to the target schema, wherein the mapping rules comprises mapping rules at a general schema level, a data record level, and a data element level of the target schema; (iv) transforming, using the derived mapping rules, at least some data of the identified source data schema to data of the target schema; (v) analyzing, using a schema validation algorithm, the data transformed to the target schema to identify any mapping anomalies; and (vi) providing, to a user via a user interface, one or more of the identified source data schema, at least some of the data transformed to the target schema, and any identified mapping anomalies.

According to an embodiment, the method further includes receiving, from the user via the user interface, feedback regarding the mapping rules and/or identified mapping anomalies, wherein based on the user's feedback the mapping rules and/or the identified mapping anomaly determination can be improved, or otherwise altered

According to an embodiment, the method further includes modifying, based on the received feedback, one or more of the mapping rules.

According to an embodiment, the method further includes updating one or more of the first trained machine learning algorithm, the second trained machine learning algorithm, and the schema validation algorithm.

According to an embodiment, one or more of the first trained machine learning algorithm and the second trained machine learning algorithm comprises a deep learning neural network.

According to an embodiment, one or more of the first trained machine learning algorithm and the second trained machine learning algorithm comprises a natural language processing neural network.

According to an embodiment, one or more of the first trained machine learning algorithm and the second trained machine learning algorithm comprises a graph neural network.

According to an embodiment, the one or more data comprises both structured and unstructured data.

According to another aspect of the invention is a system configured to map data between a source data schema and a target data schema. The system includes: one or more data sources; a normalized data set extracted from the one or more data sources; a first trained machine learning algorithm trained to analyze the normalized data set to identify the source data schema, wherein identifying the source data schema comprises identifying a general schema level, a data record level, and a data element level for the source data schema; a second trained machine learning algorithm trained to derive mapping rules configured to transform data from the identified source data schema to the target schema, wherein the mapping rules comprises mapping rules at a general schema level, a data record level, and a data element level of the target schema; a schema validation algorithm trained to analyze data transformed to the target schema to identify any mapping anomalies; a processor configured to: (i) analyze, using the first trained machine learning algorithm, the normalized data set to identify the source data schema; (ii) derive, using a second trained machine learning algorithm, mapping rules configured to transform data from the identified source data schema to the target schema; (iii) transform, using the derived mapping rules, at least some data of the identified source data schema to data of the target schema; (iv) analyze, using the schema validation algorithm, the data transformed to the target schema to identify any mapping anomalies; and a user interface configured to provide one or more of the identified source data schema, at least some of the data transformed to the target schema, and any identified mapping anomalies.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

One of the advantages of the present application is to provide a method for data sharing and data integration facilitating continued human-machine interaction and taking into account the input of the user.
Further advantage may include automatically performing changes or recommending changes for the user to consider.

Further advantage may include reporting anomalies on e.g., the mapping of data, which facilitates obtaining quality data for clinical needs.

Further advantage may include accurately validating the data and monitoring the e.g., mapping real-time or nearly real-time to ensure ongoing training of users of the system and medical personnel.

Further advantage may include data integration facilitation from various databases in view of various data sharing standards, such as HL7/FHIR, IHE, DICOM and others.
Other advantages may be envisioned.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a flowchart of a prior art method for data integration.
Fig. 2 is a flowchart of a prior art method for data integration.
Fig. 3 is a flowchart of a method for mapping data between a source data schema and a target data schema, in accordance with an embodiment.
Fig. 4 is a schematic representation of a data mapping system, in accordance with an embodiment.
Fig. 5 is a flowchart of a method for automated mapping of data from a source data schema to a target data schema, in accordance with an embodiment.
Fig. 6 is a schematic representation of a data mapping system, in accordance with an embodiment.
Fig. 7 is a schematic representation of an Al service architecture, in accordance with an embodiment.
Fig. 8 is a schematic representation of a data extraction model or process, in accordance with an embodiment.
Fig. 9 is a flowchart of feature generation, in accordance with an embodiment.
Fig. 10 is a schematic representation of schema validation and schema mapping validation, in accordance with an embodiment.
Fig. 11 is a schematic representation of the schema mapping algorithm, in accordance with an embodiment.
Fig. 12A is a user interface of the data mapping system, in accordance with an embodiment.
Fig. 12B is a user interface of the data mapping system, in accordance with an embodiment.
Fig. 13 is an example of labels for supervised training of a schema mapping algorithm, in accordance with an embodiment.
Fig. 14 is a flowchart of a method for training a machine learning algorithm of the system, in accordance with an embodiment.

### DETAILED DESCRIPTION OF EMBODIMENTS

The present disclosure describes various embodiments of a system and method configured to map data between a source data schema and a target data schema. More generally, the Applicant has recognized and appreciated that it would be beneficial to provide a method and system to automate the data integration process, including for the improved exchange of patient health data among HIT applications. Accordingly, various embodiments and implementations described or otherwise envisioned herein are directed to a method and system for automated data mapping and transformation. A data mapping system extracts and normalizes data from one or more data sources within a source data schema to generate a normalized data set. A trained machine learning algorithm of the data mapping system analyzes the extracted data to identify a source data schema. A trained machine learning algorithm of the data mapping system then derives mapping rules configured to transform data from the identified source data schema to the target schema. The data mapping system transforms at least some data of the identified source data schema to data of the target schema using the derived mapping rules. A schema validation algorithm of the data mapping system analyzes the data transformed to the target schema to identify any mapping anomalies. The data mapping system then provides the identified source data schema, at least some of the data transformed to the target schema, and/or any identified mapping anomalies to a user via a user interface. It is to be understood that a source data schema refers to a source of data (e.g., a database node in multiple nodes of databases) and refers to a data schema.

According to an embodiment, the methods and systems described or otherwise envisioned herein provides a novel and non-obvious solution for detecting and schema mapping using artificial intelligence (AI) algorithms, like deep learning algorithms. The solution addresses the challenges of data validation, integration and migration in large-scale and heterogeneous healthcare data environments.

According to an embodiment, the system comprises a data extraction module, a data schema detection module, a data mapping module, and a data validation module. The data extraction module extracts data from various sources, including healthcare system interfaces, application programming interfaces (APIs), databases, data warehouses, cloud services, and file systems, and prepares the data for schema detection and mapping. The data schema detection and mapping module applies AI techniques, including deep learning, natural language processing, and graph analysis, to characterize the data schemas. The data schema characterization AI algorithms identify the similarities and differences between each data element and a standard healthcare data schema (e.g. DICOM, HL7, etc.) or known custom data schema. Additionally, AI algorithms are used to derive mapping rules that can transform the data from the source schemas to the target schema and vice-versa. The data validation module checks the data veracity and consistency of the mapped data and provides feedback to the users for further refinement. The schema detection and mapping can be applied across the whole data set to each data element, and to each data record (collection of related data elements) to provide a general schema and to identify deviations which may occur at the element or record level. As such data schema mapping can occur on a per data element or record level, thus overcoming the impact of deviations to the general data schema.

According to an embodiment, the AI models used in the system are trained on a large and diverse set of data samples and schema models obtained from healthcare medical and data interoperability systems. Furthermore, the AI models can adapt to new data sources and schema types through a continuous learning process. The system also supports collaborative schema mapping, where multiple users can contribute their domain knowledge and feedback to improve the mapping quality.

According to an embodiment, the system can be implemented as a standalone software application, as a cloud-based service, or can be integrated with other data management tools/systems and interoperability platforms. The system can also be extended to support other data-related tasks, such as data profiling, data cleansing, data governance, and optimization of data integrations/interoperability.

Thus, according to an embodiment, AI algorithms have been developed to automate the data schema detection, mapping, and validation processes. Specifically, the process is improved by using AI-schema detection algorithms to predict the underlying data schema of the source system, quickly identifying deviations between the source and target system schemas, or the source system having a known standard or non-standard schema. Additionally, AI schema mapping algorithms predict the matching target data entity based on the source system's data entity, and AI veracity models detect anomalies between the mapped and transformed source data elements compared to the needs of the target system and apply corrected transformations as a means of validation. The algorithms are applied on a perelement basis and on the production data feed. The combination of algorithms enables the automated identification of deviations in data mapping, localizing the problem to issues with the target system schema, schema mapping, and data transformations provided as feedback to users. The result is a singlestep correction applied manually by users or automatically using the output of the AI algorithms.

The methods and systems described or otherwise envisioned herein offer solutions to numerous clinical data access and quality issues by leveraging the efficiency and robustness provided by AI algorithms. Specifically, the use of AI algorithms can reduce the complexity of manually managing data sharing and significantly reduce the time, effort, and cost required to set up and maintain data integrations through automation. By breaking down data silos, AI-powered interoperability can accelerate the integration of multiple data sources to provide a comprehensive view of patient journeys, enabling better care continuity. Moreover, AI-powered data integrations can improve data quality and veracity by ensuring accurate and contextually relevant data is shared between medical systems on a per data element basis. This approach also enables semantic interoperability, whereby data elements have additional contextual metadata and/or can be normalized to common ontology to improve their representativeness. Additionally, this methodology enables real-time analytics, operational and clinical workflow guidance, and AI algorithm development by enabling high-quality data sharing. By leveraging these benefits, healthcare organizations can significantly improve patient outcomes and drive better clinical and operational efficiencies.

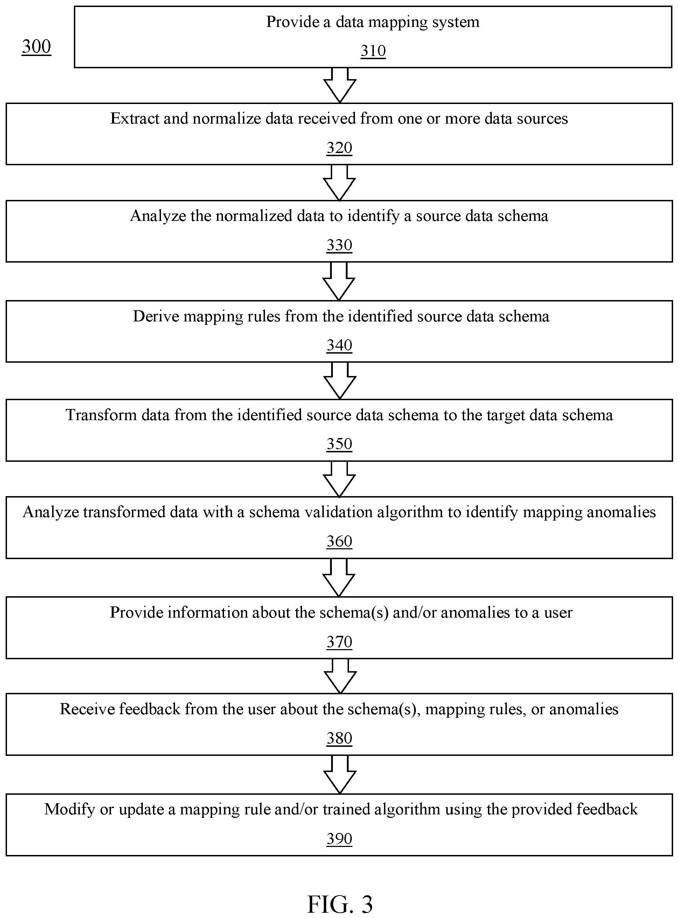

Referring to Fig. 3, in one embodiment, is a flowchart of a method 300 for mapping data between a source data schema and a target data schema using a data mapping system. The methods described in connection with the figures are provided as examples only, and shall be understood not to limit the scope of the disclosure. The data mapping system can be any of the systems described or otherwise envisioned herein. The data mapping system can be a single system or multiple different systems.

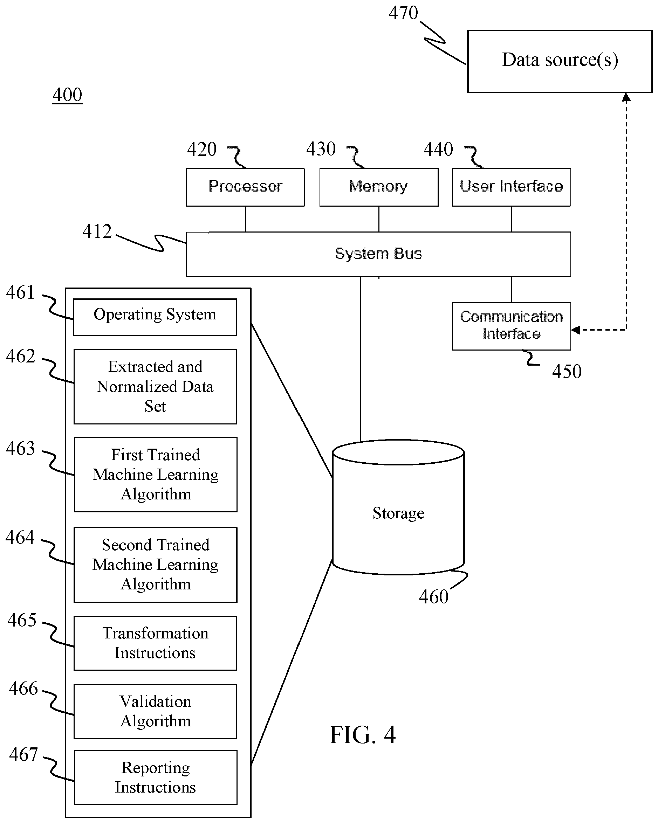

At step 310 of the method, a data mapping system 400 is provided. Referring to an embodiment of a data mapping system 400 as depicted in Fig. 4, for example, the system comprises one or more of a processor 420, memory 430, user interface 440, communications interface 450, and storage 460, interconnected via one or more system buses 412. It will be understood that Fig. 4 constitutes, in some respects, an abstraction and that the actual organization of the components of the system 400 may be different and more complex than illustrated. Additionally, data mapping system 400 can be any of the systems described or otherwise envisioned herein. Other elements and components of the data mapping system 400 are disclosed and/or envisioned elsewhere herein.

According to an embodiment, the data mapping system 400 comprises or is in communication with one or more data sources 470. In the context of healthcare data schemas, the one or more data sources are healthcare and/or healthcare-related data sources. According to an embodiment, the data sources include data and/or metadata of one or many types. For example, the data sources can include clinical, operational, meta, and other data used in a healthcare system, including electronic health records (EHRs), medical images, data from medical devices, claims data, staffing HR data patientgenerated data, and many other types and sources of data. Metadata includes, for example, data schema specifications as well as validated extract, transform, and load scripts used to train, validate, and monitor the AI models. The data can be transported using standard protocols as defined by HL7, FIHR, DICOM, rest-API, ODBC driver, amongst others, and by custom protocols. According to an embodiment, the one or more data sources 470 comprise structured and/or unstructured data including data schema, data elements, data records, and data mappings. The one or more data sources 470 may be local to the data mapping system, and may optionally be a component of the data mapping system. The one or more data sources 470 may alternatively be remote to the data mapping system, and thus is in direct or indirection communication with the data mapping system.

Thus, according to an embodiment therefore, the data mapping system 400 is a system configured for automatically detecting and mapping data schemas. The system collects, extracts, processes, and transforms data from the source and target systems. The system can be a standalone integration engine used to move data from a source system to a target system. The system may also be used within a source system, target system, or third-party integration engines.

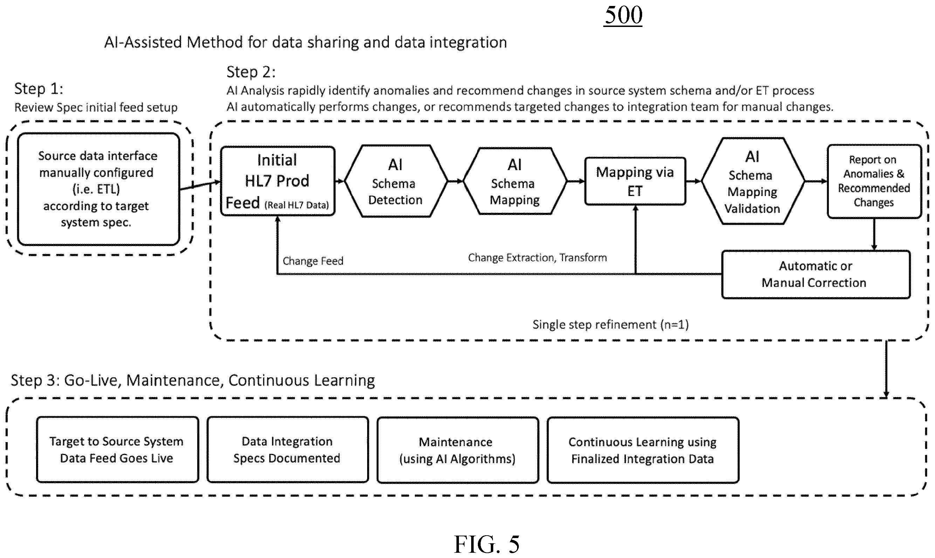

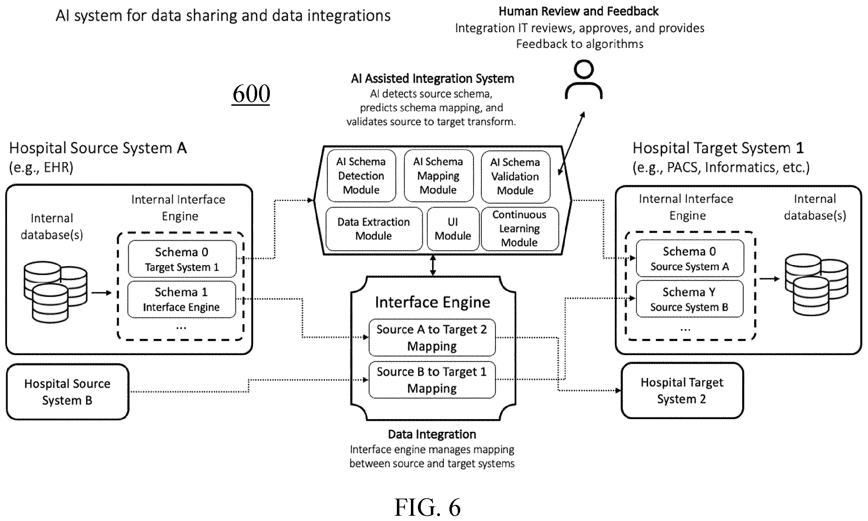

Referring to Fig. 5, in one embodiment, is a flowchart of a method 500 for automated mapping of data from a source data schema to a target data schema. Similarly, referring to Fig. 6, in one embodiment, is a schematic representation of a data mapping system implementing method 500 in Fig. 5. These methods and systems will be described in greater detail herein.

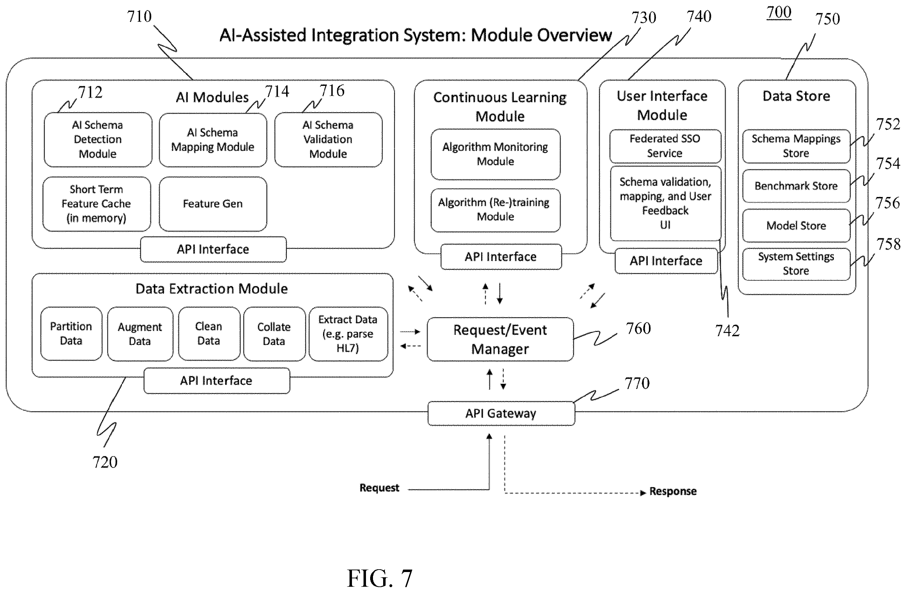

Referring to Fig. 7 is a schematic representation of an AI service architecture 700 according to an embodiment of the data mapping system. According to this embodiment, the AI service architecture 700 comprises a plurality of AI Modules 710, and a data extraction module 720 configured to partition, augment, clean, collate, and/or extract data. The AI Modules 710 include a schema detection module 712, a schema mapping module 714, and a schema validation module 716. The AI service architecture 700 also comprises a continuous learning module 730, and a user interface (UI) module 740 with a feedback module 742. Additionally, an API gateway 770 processes requests and provides responses through a standard API layer (e.g., RestAPI via POST or GET). A request/event manager 760 provides coordination of modules via appropriate routing logic for requests and responses. A data store 750 provides storage for schema mappings 752, AI algorithm model artifacts 756, AI algorithm performance benchmarks 754, and system settings 758. Other elements and components of the data mapping system 400 are disclosed and/or envisioned elsewhere herein.

Schema validation algorithms may detect anomalous data-schemas and data-elements after the source system data elements have been mapped to the target system schema (and transformed if required). Algorithms compare the post-mapped data-elements received from the source system with the expected data-schema for the target system. The AI-DIS can use the output of the schema validation algorithms to determine if there is incorrect data, new data, or missing data prior to ingesting the data into the target system. The algorithm(s) ensure a high-level of data veracity in the target system's database. Additionally, the schema mapping validation predictions can be used in combination with the schema detection and schema mapping algorithm to identify the source of erroneous or missing data, enabling automated remediation of the errors or providing feedback to humans for rapid manual correction.

Returning to method 300 in Fig. 3, at step 320 of the method, the data mapping system receives, retrieves, or otherwise obtains data from the one or more data sources 470 and extracts and normalizes the data from the one or more data sources to generate a normalized data set. According to an embodiment, the data obtained from the one or more data sources 470 comprises a source data schema. In the healthcare setting, the one or more data sources may be any healthcare and/or healthcare-related data source, such as an EHR or other data source. The data can be received, retrieved, or otherwise obtained from the one or more data sources 470 using any method for receiving data from one or a plurality of sources. Once the data is obtained, it may be utilized immediately, or it may be stored in local and/or remote memory for subsequent use by the system.

According to an embodiment, the obtained data is cleaned and normalized by a preprocessing module and converted into a machine-readable format for machine learning. Duplicates can be removed, missing data can be interpolated using imputation methods such as SMOTE, multiple imputation, and predictive models, depending on the data type. Oversampling can also be performed using standard techniques like SMOTE, although many other techniques are possible.

According to an embodiment, the extracted data can also be augmented when it is utilized for training (as described herein). Data augmentation is an advanced technique used to enhance the performance of a machine learning model by mitigating the risk of overfitting and improving the model's generalizability to new data. It entails techniques to create new samples from existing data elements, expanding the dataset's size and diversity. Here, the data augmentation can involve applying known data schema and mappings to historical data elements, generating a larger pool of realistic known data schema mappings for training the algorithms. This method effectively augments the original data by generating new data instances with different statistical properties. Additionally, transformations such as random perturbations to existing sequences of alphanumeric values and the generation of new IDs of varying length are some of the augmentations can be applied to the data. The generated data samples can be subsequently combined with the original data to create a more extensive and diverse dataset for training the machine learning model. This helps improve the model's accuracy and robustness by providing it with a more comprehensive set of representative examples of the data it is designed to classify or predict.

According to an embodiment, data can be partitioned across numerous dimensions to train context specific models. For example, HL7 data is partitioned by message type (ADT, ORU, ORM, etc) and data entity type timestamp vs alphanumeric data. According to an embodiment, a model is trained for each message type and data entity type.

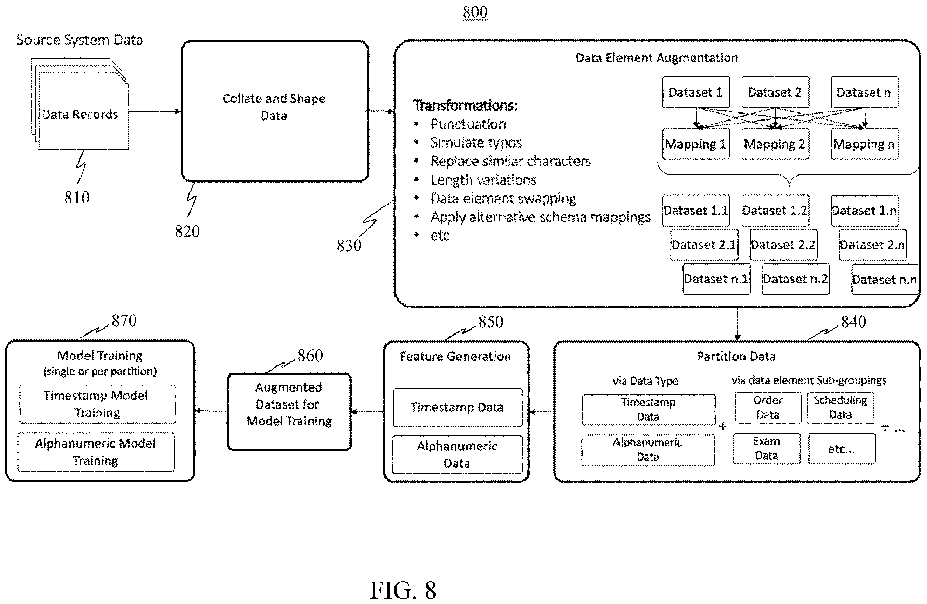

Referring to Fig. 8, in one embodiment, is a schematic representation of a data extraction model or process 800. The data extraction model or process 800 includes receiving and obtaining data from one or more data sources, such as from data records 810 which can be collated and shaped at 820. For example, referring to TABLE 1 is representative of possible data collated and organized into a data table (although other formats are possible).

**TABLE 1. Collated and Shaped Data**

| | Patent MRN | Patient Name | ... |
|---|---|---|---|
| Record1 | MRN123 | Joe | ... |
| Record2 | MRN456 | Susan | ... |
| ... | ... | ... | ... |

The data is optionally augmented at 830 to enhance the dataset. For example, the data can optionally undergo one or more transformations to improve the quality of the data, including mapping and dataset enhancement. Referring to TABLE 2, for example, are possible data transformations that enhance or augment the collated and shaped data from 820.

**TABLE 2. Data Element Transformation(s)**

| | Patent MRN | Patient Name | ... |
|---|---|---|---|
| Record1 | MRN123 | Joe | ... |
| Record1.a | MRNA54 | Joe | ... |
| Record1.b | MRNA54 | Joseph | ... |
| Record2 | MRN456 | Susan | ... |
| ... | ... | ... | ... |

Returning to Fig. 8, at 840 the data is then partitioned. The partitioning can take many different forms, including but not limited to via one or more of data type (e.g., timestamp data, alphanumeric data, etc.) and data element sub-groupings (e.g., order data, schedule data, exam data, etc.), among other possible partitions. At 850 features are generated from the partitioned data thus generating features from data types (such as timestamp data, alphanumeric data, etc.) to result in an augmented dataset 860. This augmented dataset is useful for both model training at 870, and for analysis by trained models (not shown in Fig. 8, but described or otherwise envisioned herein).

According to an embodiment, each of the AI modules comprises a feature generator, a feature cache, across and the AI model inference endpoint. Features are generated for each machine learning model type to achieve high predictive performance. The feature cache is a short-term in-memory buffer used to share features across models and enables the accumulation of data records for across record feature generation. According to an embodiment, feature generation methods are applied to different types of data, including alphanumeric, categorical, datetime, and labels.

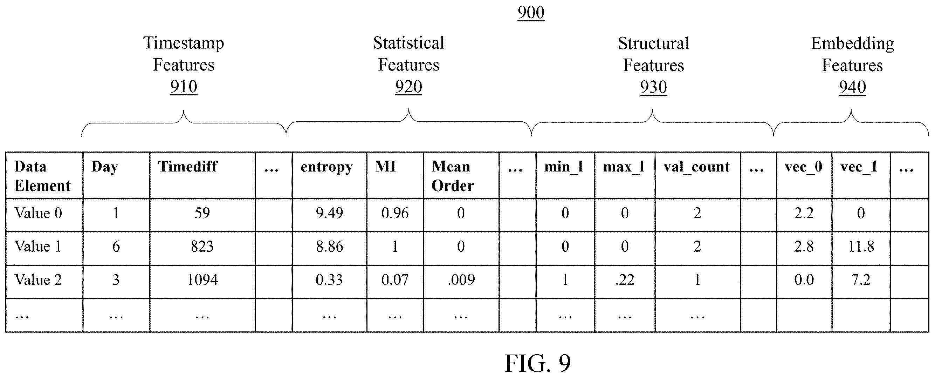

Referring to Fig. 9, for example, is a table 900 of extracted features following feature generation. According to an embodiment, the feature generation methods include one or more of Structural, Mathematical, Statistical, and Embedding features, among other possible features. Features can be calculated per data element, across data elements, or across records. TABLE 3 provides a comprehensive, but not exhaustive, list of potential features.

**TABLE 3. Potential Features**

| Timestamp Features: | |
|---|---|
| Structural representation: | Length of data element, most frequent value, number of numeric characters, number of alpha characters, etc |
| Statistical representations: | frequency, mean, median, std, entropy, mutual information, TF-IDF, et |
| Embedded representations: | Hex encoding, one-hot encoding, ordinal encoding, numeric encodings, principal component analysis, continuous bag of words, skip Grams, CNN, RNN/LSTM, Autoencoders, Transformers, generative networks, and graph neural network encodings. |

As shown in Fig. 9, a data element (such as Value 0) can be organized with Timestamp Features 910 (as a subset of the Timestamp data), as well as alphanumeric data features including Statistical Features 920, Structural Features 930, and/or Embedding features 940. As illustrative examples only, Statistical Features 920 can include entropy, mutual information (MI), mean order (n records), std order (n records), covariance, and/or correlation, among other features. Structural Features 930 can include length of data element, max length (across *n* records), min length (across *n* records, number of alpha, number of numeric, value counts of data elements, and/or order/location in a record, among other features. Embedding features 940 can include autoencoder, transformer, graph neural network, another network such as an RNN, LSTM or CNN trained using supervised learning, and/or whether output is vector numeric representation of inputs, among other features. It is to be understood that Timestamp Features is a representative example, and in some cases this might refer to sequence encoding information and different types of sequence encoding information.

At the end of step 320 of method 300 in Fig. 3, the system comprises an extracted and normalized data set 462. The extracted and normalized data set 462 may be utilized to train one or more algorithms of the system, and/or the extracted and normalized data set 462 may be utilized in subsequent steps of method 300.

At step 330 of method 300 in Fig. 3, the data mapping system 400 analyzes, using a first trained machine learning algorithm 463, the normalized data set to identify the source data schema of the one or more data sources. According to an embodiment, identifying the source data schema comprises identifying a general schema level, a data record level, and a data element level for the source data schema. It is to be understood that the general level schema might refer to a general level of a structure of a database, wherein the word "general" and "level" might refer to a related level of a database, such as a physical level, conceptual level and/or external level. The database could be of different types, namely, hierarchical databases, relational databases, non-Relational Databases, object oriented databases. It is to be understood that a data record refers to information which is processed or stored, or is to be processed, by a computer system. Data record level refers to the level the information coming from a particular database level.

According to an embodiment, the first trained machine learning algorithm 463, which can also be referred to as the schema detection module or algorithm, is any algorithm, classifier, or model capable of creating the output as described or otherwise envisioned herein, including but not limited to machine learning algorithms, classifiers, and other algorithms. The trained algorithm is a unique algorithm based on the training data used to train the algorithm. Once generated, the first trained machine learning algorithm can be utilized or deployed immediately, or it may be stored in local and/or remote memory for future use and/or deployment. Thus, the system comprises a first trained machine learning algorithm as described or otherwise envisioned herein.

According to an embodiment, the first trained machine learning algorithm is or comprises a deep learning neural network (e.g. feed forward, convolutional neural network, etc.), a statistical machine learning algorithm (e.g., XGBoost, decision tree, etc.), a natural language process network (e.g., transformer network, etc.), and/or a graph neural network (e.g., GNN, etc.), among other types of machine learning algorithms.

According to an embodiment, the first trained machine learning algorithm 463 is trained using extracted and normalized data in the extracted and normalized data set 462, although the algorithm may be trained using other data, via known methods for training a machine learning algorithm. According to an embodiment, the first trained machine learning algorithm is trained to identify a source data schema of a data source, including identifying a general schema level, a data record level, and a data element level for the source data schema.

According to an embodiment, the first trained machine learning algorithm 463 comprises one or multiple AI algorithms. The models provide schema detection for standard and non-standard schema. They can also be used to characterize unknown schemas. The algorithms predict the schema entity for each source data element and/or collection of data elements.

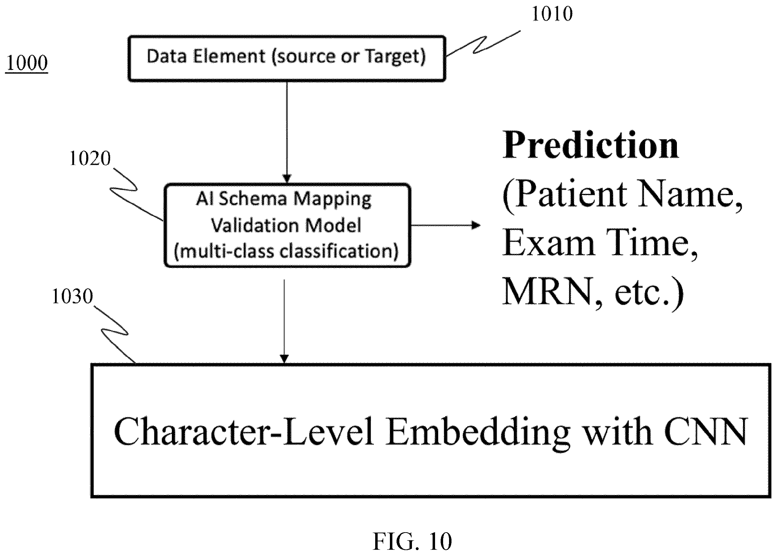

Referring to Fig. 10, in one embodiment, is a schematic representation 1000 of schema validation and schema mapping validation, using a schema mapping validation model 1020. According to an embodiment, supervised learning algorithms predict the data entity for each data element 1010 (i.e., input data which is a source or target data element) using features generated by data elements, data records, and metadata from a medical system, to generate for example a character-level embedding 1030. Algorithms include convolutional neural networks (CNN), recurrent neural networks (RNN), generative neural networks, graph neural networks (GNN), tree models, and more. In general, a supervised learning algorithm can be utilized, and the choice of algorithm can be based on optimum performance. For example, CNNs are a validated method for HL7 v2 schema validation.

According to an embodiment, for text-based data schemas (e.g. unparsed HL7 v2 message) a named entity recognition model can be used, including transformers, Bi-LSTMs, etc. Note, the Schema Mapping Validation algorithm is a specific application of Schema Detection applied to the target system post-schema-mapping.

According to an embodiment, unsupervised learning algorithms can also be used for schema detection and are useful to characterize unknown schemas. Embeddings are generated using large datasets from known schemas, using algorithms like transformers, graph neural networks, and more. The embedding algorithm is applied to data from an unknown schema and clustering, or statistical matching algorithms are used to estimate the probability of a data elements entity. Alternatively, the embedding spaces can be fine-tuned to first predict if the data match a standard schema (e.g. HL7, FHIR, DICOM, etc.), followed by the corresponding supervised algorithm for that schema to predict the data entity for each data element.

At step 340 of method 300 in Fig. 3, the data mapping system 400 derives, using a second trained machine learning algorithm 464, mapping rules configured to transform data from the identified source data schema to the target schema. According to an embodiment, mapping rules comprises mapping rules at a general schema level, a data record level, and a data element level of the target schema. In an embodiment, mapping rules are derived using machine artificial intelligence algorithms and may be based on data element augmentation and applying any one of the following transformations or combination of those: punctuation determination, replacing similar characters with other characters, length variation determination, data element swapping and others. This may have an advantage of filtering out particular type of information more efficiently.

According to an embodiment, the second trained machine learning algorithm 464, which can also be referred to as the schema mapping module or algorithm, is any algorithm, classifier, or model capable of creating the output as described or otherwise envisioned herein, including but not limited to machine learning algorithms, classifiers, and other algorithms. The trained algorithm is a unique algorithm based on the training data used to train the algorithm. Once generated, the second trained machine learning algorithm can be utilized or deployed immediately, or it may be stored in local and/or remote memory for future use and/or deployment. Thus, the system comprises a second trained machine learning algorithm as described or otherwise envisioned herein.

According to an embodiment, the second trained machine learning algorithm is or comprises a deep learning neural network (e.g. feed forward, convolutional neural network, etc.), a statistical machine learning algorithm (e.g., XGBoost, decision tree, etc.), a natural language process network (e.g., transformer network, etc.), and/or a graph neural network (e.g., GNN, etc.), among other types of machine learning algorithms.

According to an embodiment, the second trained machine learning algorithm 464 is trained using extracted and normalized data in the extracted and normalized data set 462, although the algorithm may be trained using other data, via known methods for training a machine learning algorithm. According to an embodiment, the second trained machine learning algorithm is trained to derive mapping rules configured to transform data from the identified source data schema to the target schema.

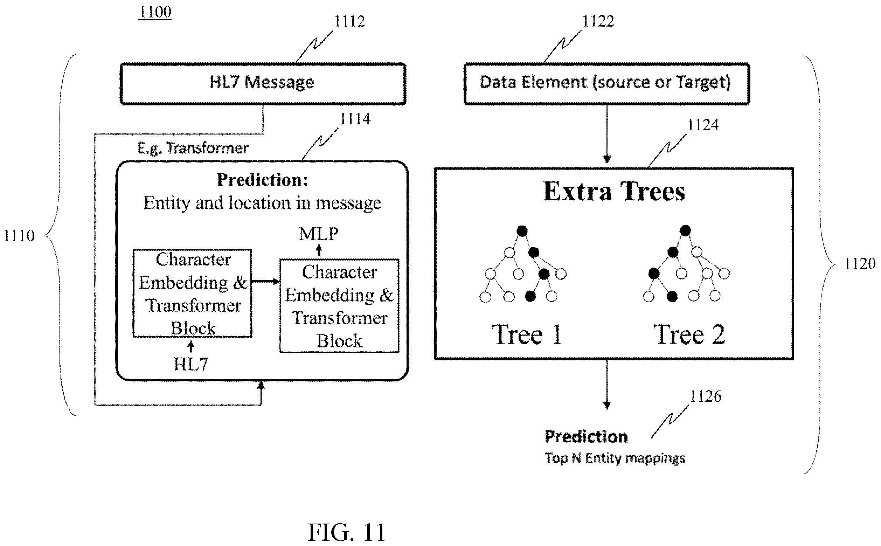

Referring to Fig. 11, in one embodiment, is a schematic representation 1100 of a schema mapping algorithm overview. According to an embodiment, the second trained machine learning algorithm 464 comprises one or multiple AI algorithms. The model(s) provide schema mapping for standard and non-standard schema, and can also be utilized to map unknown schemas. The algorithm(s) predict the target system entity for each source system data element or collection of source data elements (e.g. a column of data in a structured table based schema).

According to an embodiment, a model trained by supervised a learning algorithm predicts the data entity for each data element using features generated by data elements, data records, and metadata from a medical system. Possible algorithms include convolutional neural networks (CNN), recurrent neural networks (RNN), generative neural networks, graph neural networks (GNN), tree models, and more. Algorithm selection is performed by optimizing predictive performance for different schema, data partitions, and so on.

For text-based data schemas (e.g., unparsed HL7 v2 message) a named entity recognition model can be used, including transformers, Bi-LSTMs, etc. Additionally, these types of schema mapping algorithms based on transformers and Bi-LSTMs, can be trained to predict the transformation in addition to the correct entity in the target system schema.

According to an embodiment, unsupervised learning algorithms can also be used for schema mapping. Embedding spaces are generated using large datasets from known source and target systems schemas. Possible algorithms include transformers, graph neural networks, and more. Clustering, statistical matching, and/or a supervised learning algorithms are used to map the source system data elements to the target system elements based on similarity of embedding spaces. In the case of a supervised learning, it is applied post embedding and trained to predict whether a source data element matches a data element using. For example, training a generative adversarial network, transformers, or other similar networks to predict match versus no match.

Thus, in Fig. 11, at 1110, a model can be used for text-based data schemas (e.g., unparsed HL7 v2 message 1112), including transformers, Bi-LSTMs, and so on. A schema mapping algorithm 1114 based on a transformer or Bi-LSTM can be trained, for example, to predict the transformation in addition to the correct entity in the target system schema. At 1120, a data element (source or target) 1122 is input to a model such as a tree-based model 1124, for a prediction 1126.

At step 350 of method 300 in Fig. 3, the data mapping system 400 transforms, using the mapping rules derived by the second trained machine learning algorithm 464, data from the identified source data schema to data of the target schema. The transformed data may be any data that is organized pursuant to the identified source data schema. For example, the data to be transformed may be some or all of the normalized data set extracted from the one or more data sources 470. Additionally or alternatively, the data to be transformed may be new data obtained from the one or more data sources 470 or obtained from another source that uses the identified source data schema. The amount of data transformed by the system may be any amount of data. Once the data is transformed by the system, it may be utilized immediately, or it may be stored in local and/or remote memory for subsequent use by the system.

At step 360 of method 300 in Fig. 3, the data mapping system 400 analyzes, using a schema validation algorithm 466, some or all of the data transformed from the identified source data schema. to the target schema. According to an embodiment, the schema mapping validation algorithm is a similar in application and design with the schema detection algorithms. For schema validation the algorithms are trained to predict the target system using post-schema-mapping data elements as input.

At step 370 of method 300 in Fig. 3, the data mapping system 400 provides, using a user interface of the system, one or more of the identified source data schema, at least some of the data transformed to the target schema, and any identified mapping anomalies. Other information relevant to the system may also be provided. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands. According to an embodiment, the display may further comprise many other types of information.

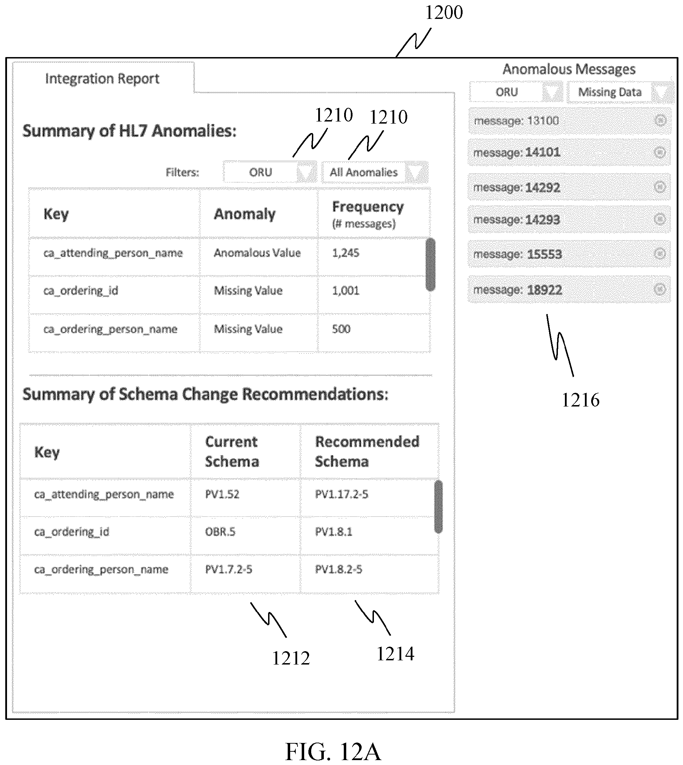

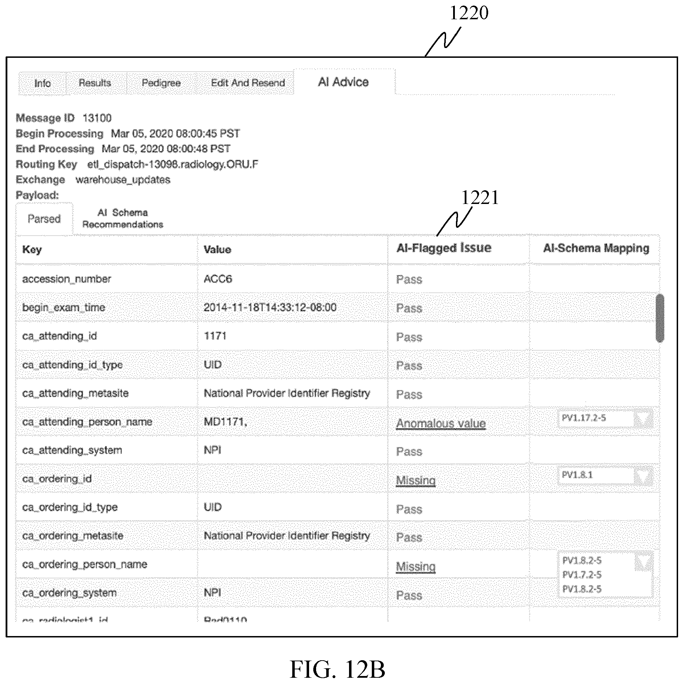

Referring to Figs. 12A and 12B are possible embodiments of a user interface of the data mapping system 400. According to an embodiment, the user interface/user experience (UI/UX) module provides an interactive session to view AI recommendations, manually perform an integration, and/or provide user feedback on the AI predictions. For example, the system can allow users to agree or disagree (or abstain if unsure) with a mapping rule or prediction, as well as provide explicit corrections to the AI model's mapping or predictions in the case of disagreement. The feedback is gathered through a standalone user-interface that prompts the user to provide their feedback, which can also be integrated into the schema validation/mapping workflow when validating ETL scripts.

Referring to Fig. 12A is an integration report 1200, which can be a component of a display via the user interface of the data mapping system. The integration report can provide a summary of issues, such as HL7 anomalies and anomalous messages. For example, a list of problematic HL7 messages and data elements are provided ("Summary of HL7 Anomalies"), with filtering capabilities 1210 enabling the user to quickly dive into the anomalies, errors, or missing data, among other possible filters. Anomalous messages can be identified and/or sorted via filters at 1216. The integration report can also provide a summary of schema change recommendations identified by the system, with side-by-side comparison of source schema ("Current Schema" 1212) and target schemas ("Recommended Schema" 1214), with lines connecting the mapped elements.

According to an embodiment, incorrect source schema data elements can be corrected by the user via the source system. The user can rerun each of the AI algorithms and quickly review previously erroneous HL7 message/data elements to ensure accuracy resulting from the correction and rerun. The user performs acceptance testing by finalizing and approving the schema mapping.

Alternatively, incorrect or suboptimal schema mappings also modified by user through the UI system. For example, the user can select an anomalous message in the UI interface shown in Fig. 12A (here, anomalous message "message: 13100" is selected and the message is displayed in Fig. 12B as 1220). The message shows data elements line by line, with AI-flagged issues (such as missing or anomalous values in the "AI-Flagged Issue" column 1222), with the corresponding AI-detected schema mapping listed. The user may agree with the schema prediction (such as by selecting or saving a schema prediction). The user may alternatively disagree with the prediction, either by indicating disagreement or by selecting or identifying a different schema or schema prediction. The user interface can prompt the user to provide some or all of the necessary information.

The collected feedback is analyzed to identify patterns and trends, which can help to determine the areas of the model that require improvement and the necessary changes required to improve accuracy and performance. For instance, feedback may highlight specific database entities, data types (such as datetimes), HL7 message types, groups of records, and other aspects that require attention.

Based on the feedback analysis, changes can be made to the appropriate AI model's architecture, features, training methods, data inputs, or other aspects of the machine learning process to improve performance. Alternatively, the changes can be directly incorporated into the model through continuous learning as described herein.

### ALGORITHM TRAINING

The schema detection algorithm (first trained machine learning algorithm 463), the schema mapping algorithm (second trained machine learning algorithm 464), and the validation algorithm 466 can be trained using standard machine learning optimization approaches, hyperparameter optimization, cross-validation, hold-out test sets, and so on. Individual algorithms can be trained per partitions of augmented data. Additionally, multiple algorithms can be trained independently or end to end to predict different aspects of a data schema, including predicting the entity of a data element, the type of data record (e.g.. HL7 message type or segment), predicting an unknown schema matches specific standard schema (e.g.. HL7, FHIR, or DICOM), and predicting the specific transformation performed during schema mapping (e.g., merging of multiple source system data elements into a single target system data element, formatting a data elements, etc.). Additionally, a model can be local, or available through federated learning whereby multiple local or cloud-hosted source/target systems create independent AI models that are federated into one performant model without the need to share data across systems. For supervised learning, labels are extracted from the schema mapping script whereby the target system data entity. For unsupervised learning, no labels are required, instead embeddings are generated.

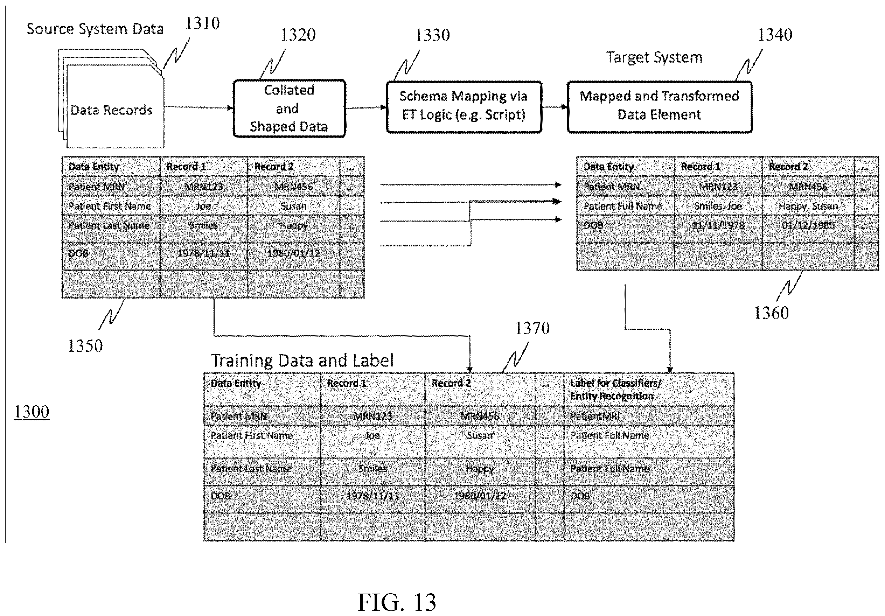

Referring to Fig. 13, for example, is an example 1300 of labels extracted from a schema mapping script, for supervised training of the schema mapping algorithm 464. As described in conjunction with Fig. 8, a data extraction model or process includes receiving and obtaining data from one or more data sources, such as from data records 1310 which can be collated and shaped at 1320. For example, table 1350 in Fig. 13 can be collated into table 1360. For example, the data can optionally undergo one or more transformations to improve the quality of the data, including mapping and dataset enhancement. Schema mapping of the collated and shaped data can be processed at 1330, to generate mapped and transformed data elements 1340. This dataset is useful for both model training and for analysis. Table 1370, for example, comprises extracted training data, collated with a label to be used for a classifier and/or entity recognition training.

According to an embodiment, a model can be validated by testing its predictive performance (e.g., F1-score, ROC-AUC, accuracy, etc.) using cross-validation on a hold-out data-set and estimated test performance on a new dataset. Data augmentation and continuous learning can be used to ensure that the models generalize well to new data and schemas.

Once trained, an algorithm may be deployed either as a cloud native hosted service or as a self-managed on-premises service as a containerized service. The algorithms can be referenced via an API, such as through a central API gateway provided by the system.

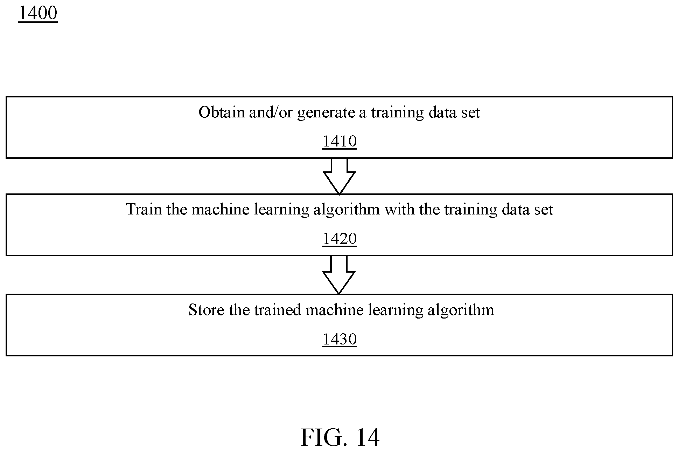

Referring to Fig. 14, in one embodiment, is a flowchart of a method 1400 for training one or more of the schema detection algorithm (first trained machine learning algorithm 463), the schema mapping algorithm (second trained machine learning algorithm 464), and the validation algorithm 466. The method may be performed by the data mapping system 400, or may be performed by another system configured to train machine learning algorithms.

At step 1410 of the method, the system tasked with training obtains or receives training data comprising data from one or more sources (such as one or more data sources 470 among many other possible sources) that is organized pursuant to a source schema. This training data may thus be obtained from or stored in a database or system or component of the data mapping system or a training system, or may be obtained from or stored in a database or system that is in direct or indirect communication with the data mapping system or a training system.

According to an embodiment, the training system may comprise a data pre-processor or similar component or algorithm configured to process the received training data. For example, the data pre-processor analyzes the training data to remove noise, bias, errors, and other potential issues. The data pre-processor may also analyze the input data to remove low quality data. Many other forms of data preprocessing or data point identification and/or extraction are possible.

For example, as described herein, the data may be augmented. According to an embodiment, data augmentation is an advanced technique used to enhance the performance of a machine learning model by mitigating the risk of overfitting and improving the model's generalizability to new data. It entails techniques to create new samples from existing data elements, expanding the dataset's size and diversity. Data augmentation can involve applying known data schema and mappings to historical data elements, generating a larger pool of realistic known data schema mappings for training the algorithms. This method effectively augments the original data by generating new data instances with different statistical properties. Additionally, transformations such as random perturbations to existing sequences of alphanumeric values and the generation of new IDs of varying length are some of the augmentations are applied to the data. The generated data samples are subsequently combined with the original data to create a more extensive and diverse dataset for training the machine learning model. This helps improve the model's accuracy and robustness by providing it with a more comprehensive set of representative examples of the data it is designed to classify or predict.

At step 1420 of the method, the machine learning algorithm is trained using the training dataset comprising the obtained data. The algorithm may be the schema detection algorithm (first trained machine learning algorithm 463), the schema mapping algorithm (second trained machine learning algorithm 464), and/or the validation algorithm 466. For example, the schema detection algorithm is trained, using training data, to identify a source schema from data. The schema mapping algorithm is trained, using training data, to generate mapping rules to map data from a source schema to a target schema. The validation algorithm is trained to analyze transformed data in order to identify mapping anomalies. According to an embodiment, the models are trained using the training data set according to known methods for training a model. Following training, the system comprises a trained schema detection algorithm 463, a trained schema mapping algorithm 464, and a trained validation algorithm 466.

At step 1430 of the method, the trained algorithm is stored for future use. According to an embodiment, the trained algorithm may be stored in local or remote storage.

### CONTINUOUS LEARNING

Referring to step 380 of method 300 in Fig. 3, the user may provide feedback or input to the data mapping system or a machine learning algorithm training system via the user interface in response to the provided information from step 370 of the method. For example, the feedback may take many forms, and may for instance be feedback regarding the mapping rules and/or any identified mapping anomalies, if there are any.

Thus, at step 190 of the method, the mapping system may modify, based on the received feedback, one or more of the mapping rules. Similarly, the mapping system may update one or more of the trained schema detection algorithm 463, trained schema mapping algorithm 464, and/or trained validation algorithm 466 based on the user input or based on new or updated training data.

According to an embodiment, the system may comprise a continuous learning module that provides the ability to monitor and continuously train the algorithms over time to ensure consistent and generalizable performance. The module captures metrics that include feedback from users via the UI module (such as discordance between a model and a human providing feedback), data quality metrics (e.g., data drift or target drift), and algorithm performance metrics (e.g., F1 score), which are used for further training of the algorithm. The module can also be used to track KPI's on the integration performance, including time for integration, number of and time spent correcting AI predictions, number of iterations to correct the source data schema, and more. The monitoring module can trigger retraining either automatically or manually after a critical performance threshold and/or integration performance KPI has been violated (e.g. F1 < 0.9).

Continuous learning can be accomplished using three approaches, including: (1) retrain the entire model after every new integration is complete; (2) fine-tuning the model after every new integration; or (3) retraining based performed using a human-in-the-loop reinforcement learning (RL) method. For the first two methods, the final schema mapping (or detection, validation) data is used to update the algorithms. The human-in-the-loop reinforcement learning method requires incorporating an RL agent with the supervised learning model. Human feedback includes agreeing, disagreeing, or abstaining from a prediction. In the case of disagreement, the human can provide the correct outcome through the UI module. The human feedback is used to provide a reward, penalty, or neutral direct to the RL agent, which in turn is used to guide the supervised learning process. For example, using a proximal policy optimization (PPO) to fine-tune the algorithm.

Continuous learning can improve the accuracy and reliability of a model, ensuring that it remains effective in predicting database schemas. The continuous learning module manages model retraining when supplied with new data and labels. The new data and labels may be supplied from integration data from historically performed data integrations or new integration data from manually completed data integrations, or from human feedback. The model can be retrained either manually or automatically. The model's performance is validated using online or offline A/B testing on a small subset of data. If the retrained model's performance is maintained or improved, as evaluated using standard metrics such as precision, recall, and F1 score, it is moved to production.

### EXAMPLES

The following are provided only as possible embodiments, implementations, or examples of the methods and systems described or otherwise envisioned herein. Accordingly, it is understood that these examples do not limit the scope of the methods and systems described or otherwise envisioned herein.

According to an embodiment, the methods and systems described or otherwise envisioned herein provide for the seamless integration of data between source and target systems in various configurations. The methods and systems are used to adapt the source schemas, as well as for extract and transform processes of the target schema to ensure efficient data-integration and accurate data transfer. The system is designed to provide a single phase, single iteration data integration between a source and a target system. The goal is to improve upon the current manual process which consists of multi-phase (test and production), and multiple iterations (user acceptance testing for nearly each data element in the source system).

According to an embodiment, the system can be implemented in three distinct configurations, as detailed below.

### 1. Incorporated into a source system.

According to an embodiment, the data mapping system is incorporated into a source system. In this case the target system must provide data to the source system on the required source and target schema specifications. The integrated data mapping system processes the source systems data, aligns, and maps it to the target systems schema using the target system specifications. The schema detection validation algorithm validates the source systems schema against the specification requested by the target system. The schema mapping is used to map the source and target schemas. The source system schema is mapped automatically to correspond with the target system specifications. The schema validation algorithm provides acceptance testing.

A benefit of this approach is that the data mapping system solution can be configured to automatically change (via ET) the source systems schema should any schema entities be missing.

### 2. Incorporated into a target system.

According to an embodiment, the data mapping system is incorporated into the target system. In this case the source system must provide data to the target system on the source system's schema specification. The integrated data mapping system processes the source systems data, aligns, and maps it to the target systems schema using the target system specifications. The schema validation algorithm validates the source systems schema against the specification required by the target system. The schema mapping algorithm is used to map the source and target schemas. The source system schema is mapped automatically to correspond with the target system specifications. The schema validation algorithm provides acceptance testing. A benefit of this approach is that there is no need to share data with the source system to perform schema detection and schema mapping validation. Another benefit is that the target system can be used to train the algorithms using all the past integrations performed, ensuring high generalizability for integration and high data veracity.

### 3. Standalone system or incorporated into a central integration engine.

According to an embodiment, the data mapping system is a standalone system that is connected to the target and source systems. In this case both the source and target systems provide data the central data mapping system service. The integrated data mapping system processes the source systems data, aligns, and maps it to the target systems schema using the target system specifications. The schema validation algorithm validates the source systems schema against the specification required by the target system. The schema mapping is used to map the source and target schemas. The source system schema is mapped automatically to correspond with the target system specifications. The schema validation provides acceptance testing. A benefit of this approach is that the data mapping system can be used as a hub-and spoke model facilitating data-integrations between numerous source and target systems. This data enables algorithms to be trained in a manner that ensures a high level of generalizability and high data veracity. Additionally, the data mapping system may be able to manage missing data in the source feed by searching across all source feeds for all integrations.

According to an embodiment, the integration workflow can be automated using modules along with UI module to view, provide feedback on, or bypass and change predictions made by algorithms performing schema mapping. The workflow involves a manual upload or connection from the source system to the target data system using production data. The schema detection algorithms are first applied to detect and validate the source schema, the schema mapping algorithms are then applied to predict the mapping between source and target data entities, and finally the schema mapping validation algorithms are applied to ensure only high veracity data is ingested into the target systems database. The UI mode can be used to provide information about the schema mappings in a visual format, allowing for humans to rapidly review the algorithm-generated predictions on a per data element, data record, and data element level. The visualization provides a side-by-side comparison of source and target schemas, with lines connecting the mapped elements. The UI modules also the user to quickly hone into the specific data elements, records, or entities whereby anomalies, errors, or missing data impacts the schema mapping, along with the predicted mappings. Incorrect or suboptimal schema mappings can be modified by the user through the UI system. This may involve changing the source schema data to include missing data, to modify the entity relationships between the source and target schemas, by adding new mappings, by removing incorrect ones, or modifying data element transformations. Additionally, the user can create a schema mapping manually, bypassing the algorithm-generated predictions altogether. This might be necessary for complex cases where the algorithm struggles to accurately predict the correct mappings. The schema mappings can be configured for automated approval based on specific KPIs for data consistency and veracity between the source and target schemas, or they can be validated by humans if required. The post validated schema mapping can be automatically or manually implemented. This step may involve data migration, transformation, or integration processes. Users can monitor the results of the schema mapping process and make any necessary adjustments to improve the accuracy and efficiency of the mappings over time. The algorithm can learn from user feedback and modifications, ultimately enhancing its performance in future schema mapping tasks.

According to an embodiment, the algorithms provide a prediction of the underlying data schema of the source system. The schema alignment happens on a per-data-element basis, providing an entity for each data element. The output of the algorithm(s) are automatically compared with the expected schema of the target system, and the mismatches in schema identified. differences between the source and target system can be automatically documented and corrected for during the schema mapping step. Alternatively, the source schema interface can be manually or automatically adjusted through the feedback module to correct the source schema in a single step to match the that of the target system. The latter is required is the source schema is missing data entities. Automatic adjusted requires incorporation of the system or API connectivity between the data mapping system and the source system that enables the data mapping system to search for the missing schema entities in the source systems database.

The schema mapping provides automated schema mapping between a source and targeting systems by predicting or statistically matching the target data entity based on the source systems data schema, data element(s) and data record(s). The schema mapping predictions can be performed in real-time by continuously applying the schema mapping algorithm to the source feed. Alternatively, the schema mapping algorithms can automatically create an ET script during the initial integration. The algorithms (and script) can be updated at pre-configured intervals, when a performance degradation on new data has been detected, or through manual human intervention. The schema mapping predictions are available via the user interface module for review, acceptance, and/or manual correction by a human.

Schema validation algorithms detect anomalous data-schemas and data-elements after the source system data elements have been mapped to the target system schema (and transformed if required). Algorithms compare the post-mapped data-elements received from the source system with the expected data-schema for the target system. The data mapping system can use the output of the schema validation algorithms to determine if there is incorrect data, new data, or missing data prior to ingesting the data into the target system. The algorithm(s) ensure a high-level of data veracity in the target system's data base. Additionally, the schema mapping validation predictions can be used in combination with the schema detection and schema mapping algorithm to identify the source of erroneous or missing data, enabling automated remediation of the errors or providing feedback to humans for rapid manual correction.

### EXAMPLE: HL7 Version 2 Integration

According to one possible embodiment, a specific use case is the Health Level Seven International (HL7) integration between electronic medical record (EMR) software and an informatics solution. Here the EMR is the source system, and the informatics system is the target system. HL7 is a standard used for the exchange, integration, sharing, and retrieval of electronic health information. HL7 Version 2 (V2) is a widely used messaging standard in healthcare that defines the structure and encoding of messages exchanged between healthcare systems. Below are the steps for using the data mapping system to facilitate an HL7 V2 integration, in accordance with an embodiment. It is assumed that the data mapping system is configured as a standalone system connected to a standard HL7 interface engine and is not configured to automatically correct the source data schema.

### 1. Data Feed Setup

Establish a production feed consisting of various HL7 V2 message types (e.g., ADT, ORM, ORU), and is connected to an interface engine. Connectivity occurs via the Minimal Lower Layer Protocol (MLLP) over TCP/IP. The interface is responsible for receiving, parsing, calling the AI-DIS for schema mapping, and routing HL7 messages between the connected systems.

### 2. Schema Detection

The schema detection algorithm(s) is trained to predict the source schema specification required by the target system to ensure all data elements are present and have high veracity. The algorithms take the parsed HL7 data elements as input (from the product feed data schema) and predict the corresponding data entity. The predictions are provided as a report out on schema deviations, data element errors, and missing data via the UI module.

### 3. Schema Mapping

The schema mapping algorithm(s) is trained to predict the target system data entity each source system data element. The data elements are parsed from the HL7 message. An algorithm is trained for each message type, and separate feature generation approaches are used for timestamp and alphanumeric data elements. The top-3 predicted of schema mappings overall and for each data element is report via the UI module.

### 4. Schema Mapping Validation

The schema validation algorithm(s) is trained to predict the target system data entity for each post-schema mapped and transformed data element. The predictions are provided as a report out on source system data element deviations, data entity errors, data element errors, and missing data via the UI module.

### 5. User Feedback and Acceptance

The schema detection, schema mapping, and schema mapping validation predictions are presented via the UI. The UI provides a report out on issues with the source schema and schema mapping. A side-by-side comparison of source and target schemas, with lines connecting the mapped elements is presented. A list of problematic HL7 messages and data elements are provided, with filtering capabilities enabling the user to quickly dive into the anomalies, errors, or missing data. Incorrect source schema data elements are corrected by the user via the source system. Incorrect or suboptimal schema mappings also modified by user through the UI system. The user reruns each of the AI algorithms and quickly reviews previously erroneous HL7 message/data elements to ensure correctness. The user performs acceptance testing by finalizing and approving the schema mapping.

### 6. Go-Live and Maintenance

For simple schema mappings (e.g., schema mappings required only a source to target data entity mapping for all HL7 messages) the interface engine is used to apply the schema mapping using the approved schema mapping script produced by the data mapping system. For complex mappings (e.g. schema mappings required per data element or per HL7 message), the interface engine uses the data mapping system provide the mapping in continuously and in real-time. Maintenance occurs by continuously monitoring the output of the source schema, the schema mapping, and schema validation algorithms and alerting the user to deviations in schema mapping. Deviations in schema mappings are detected as changes in the source schema, as detected by the schema detection algorithms, and deviations in the post-mapping data elements detected by the schema mapping validation algorithms. The user is alerted in real-time of the changes and can quickly review and remedy the issues via the UI.

### 7. Continuous Learning

User feedback during the initial integration and during maintenance are used to update the algorithms. Additionally, degradation of each algorithm's performance is monitored periodically. The user has the option to configure the system to automatically train the algorithms if a correction to the schema has been detected or if the algorithm performance has degraded beyond a pre-determine level (e.g., F1-score below 0.9). Alternatively, the user can manually trigger re-training as needed.

Referring to Fig. 4 is a schematic representation of a data mapping system 400. System 400 may be any of the systems described or otherwise envisioned herein, and may comprise any of the components described or otherwise envisioned herein. It will be understood that Fig. 4 constitutes, in some respects, an abstraction and that the actual organization of the components of the system 400 may be different and more complex than illustrated.

According to an embodiment, system 400 comprises a processor 420 capable of executing instructions stored in memory 430 or storage 460 or otherwise processing data to, for example, perform one or more steps of the method. Processor 420 may be formed of one or multiple modules. Processor 420 may take any suitable form, including but not limited to a microprocessor, microcontroller, multiple microcontrollers, circuitry, field programmable gate array (FPGA), application-specific integrated circuit (ASIC), a single processor, or plural processors.

Memory 430 can take any suitable form, including a non-volatile memory and/or RAM. The memory 430 may include various memories such as, for example L1, L2, or L3 cache or system memory. As such, the memory 430 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 400. It will be apparent that, in embodiments where the processor implements one or more of the functions described herein in hardware, the software described as corresponding to such functionality in other embodiments may be omitted.

User interface 440 may include one or more devices for enabling communication with a user. The user interface can be any device or system that allows information to be conveyed and/or received, and may include a display, a mouse, and/or a keyboard for receiving user commands. In some embodiments, user interface 440 may include a command line interface or graphical user interface that may be presented to a remote terminal via communication interface 450. The user interface may be located with one or more other components of the system, or may located remote from the system and in communication via a wired and/or wireless communications network.

Communication interface 450 may include one or more devices for enabling communication with other hardware devices. For example, communication interface 450 may include a network interface card (NIC) configured to communicate according to the Ethernet protocol. Additionally, communication interface 450 may implement a TCP/IP stack for communication according to the TCP/IP protocols. Various alternative or additional hardware or configurations for communication interface 450 will be apparent.

Storage 460 may include one or more machine-readable storage media such as read-only memory (ROM), random-access memory (RAM), magnetic disk storage media, optical storage media, flash-memory devices, or similar storage media. In various embodiments, storage 460 may store instructions for execution by processor 420 or data upon which processor 420 may operate. For example, storage 460 may store an operating system 461 for controlling various operations of system 400.

It will be apparent that various information described as stored in storage 460 may be additionally or alternatively stored in memory 430. In this respect, memory 430 may also be considered to constitute a storage device and storage 460 may be considered a memory. Various other arrangements will be apparent. Further, memory 430 and storage 460 may both be considered to be non-transitory machine-readable media. As used herein, the term non-transitory will be understood to exclude transitory signals but to include all forms of storage, including both volatile and non-volatile memories.

While system 400 is shown as including one of each described component, the various components may be duplicated in various embodiments. For example, processor 420 may include multiple microprocessors that are configured to independently execute the methods described herein or are configured to perform steps or subroutines of the methods described herein such that the multiple processors cooperate to achieve the functionality described herein. Further, where one or more components of system 400 is implemented in a cloud computing system, the various hardware components may belong to separate physical systems. For example, processor 420 may include a first processor in a first server and a second processor in a second server. Many other variations and configurations are possible.

According to an embodiment, system 400 comprises or is in direct or indirect communication with one or more data sources 470. In the context of healthcare data schemas, the one or more data sources are healthcare and/or healthcare-related data sources. According to an embodiment, the data sources include data and/or metadata of one or many types. For example, the data sources can include clinical, operational, meta, and other data used in a healthcare system, including electronic health records (EHRs), medical images, data from medical devices, claims data, staffing HR data patientgenerated data, and many other types and sources of data. Metadata includes, for example, data schema specifications as well as validated extract, transform, and load scripts used to train, validate, and monitor the AI models. The data can be transported using standard protocols as defined by HL7, FIHR, DICOM, rest-API, ODBC driver, amongst others, and by custom protocols. According to an embodiment, the one or more data sources 470 comprise structured and/or unstructured data including data schema, data elements, data records, and data mappings. The one or more data sources 470 may be local to the data mapping system, and may optionally be a component of the data mapping system. The one or more data sources 470 may alternatively be remote to the data mapping system, and thus is in direct or indirection communication with the data mapping system.

According to an embodiment, storage 460 of system 400 may store one or more algorithms, modules, and/or instructions to carry out one or more functions or steps of the methods described or otherwise envisioned herein. For example, storage 460 may comprise, among other instructions or data, an extracted and normalized data set 462, a schema detection algorithm (first trained machine learning algorithm 463), a schema mapping algorithm (second trained machine learning algorithm 464), transformation instructions 465, a validation algorithm 466, and reporting instructions 467.

According to an embodiment, the system comprises an extracted and normalized data set 462 which can be utilized to train one or more algorithms of the system, and/or can be utilized according to method 300 in Fig. 3. According to an embodiment, the data mapping system receives, retrieves, or otherwise obtains data from the one or more data sources 470 and extracts and normalizes the data from the one or more data sources to generate a normalized data set. According to an embodiment, the data obtained from the one or more data sources 470 comprises a source data schema. The data can be received, retrieved, or otherwise obtained from the one or more data sources 470 using any method for receiving data from one or a plurality of sources. Once the data is obtained, it may be utilized immediately, or it may be stored in local and/or remote memory for subsequent use by the system.

According to an embodiment, the schema detection algorithm (first trained machine learning algorithm 463) is trained to analyze a data set to identify the source data schema of the one or more data sources. According to an embodiment, identifying the source data schema comprises identifying a general schema level, a data record level, and a data element level for the source data schema. According to an embodiment, the first trained machine learning algorithm 463, which can also be referred to as the schema detection module or algorithm, is any algorithm, classifier, or model capable of creating the output as described or otherwise envisioned herein, including but not limited to machine learning algorithms, classifiers, and other algorithms. The trained algorithm is a unique algorithm based on the training data used to train the algorithm. Once generated, the first trained machine learning algorithm can be utilized or deployed immediately, or it may be stored in local and/or remote memory for future use and/or deployment. Thus, the system comprises a first trained machine learning algorithm as described or otherwise envisioned herein. According to an embodiment, the first trained machine learning algorithm is or comprises a deep learning neural network (e.g. feed forward, convolutional neural network, etc.), a statistical machine learning algorithm (e.g., XGBoost, decision tree, etc.), a natural language process network (e.g., transformer network, etc.), and/or a graph neural network (e.g., GNN, etc.), among other types of machine learning algorithms.

According to an embodiment, the schema mapping algorithm (second trained machine learning algorithm 464) is trained to derive mapping rules configured to transform data from the identified source data schema to the target schema. According to an embodiment, mapping rules comprises mapping rules at a general schema level, a data record level, and a data element level of the target schema. According to an embodiment, the second trained machine learning algorithm 464, which can also be referred to as the schema mapping module or algorithm, is any algorithm, classifier, or model capable of creating the output as described or otherwise envisioned herein, including but not limited to machine learning algorithms, classifiers, and other algorithms. The trained algorithm is a unique algorithm based on the training data used to train the algorithm. Once generated, the second trained machine learning algorithm can be utilized or deployed immediately, or it may be stored in local and/or remote memory for future use and/or deployment. Thus, the system comprises a second trained machine learning algorithm as described or otherwise envisioned herein. According to an embodiment, the second trained machine learning algorithm is or comprises a deep learning neural network (e.g. feed forward, convolutional neural network, etc.), a statistical machine learning algorithm (e.g., XGBoost, decision tree, etc.), a natural language process network (e.g., transformer network, etc.), and/or a graph neural network (e.g., GNN, etc.), among other types of machine learning algorithms.

According to an embodiment, the transformation instructions 465 direct the system to transform, using the mapping rules derived by the second trained machine learning algorithm 464, data from the identified source data schema to data of the target schema. The transformed data may be any data that is organized pursuant to the identified source data schema. For example, the data to be transformed may be some or all of the normalized data set extracted from the one or more data sources 470. Additionally or alternatively, the data to be transformed may be new data obtained from the one or more data sources 470 or obtained from another source that uses the identified source data schema. The amount of data transformed by the system may be any amount of data. Once the data is transformed by the system, it may be utilized immediately or it may be stored in local and/or remote memory for subsequent use by the system.

According to an embodiment, the validation algorithm 466 is trained to analyze some or all of the data transformed from the identified source data schema. to the target schema. According to an embodiment, the schema mapping validation algorithm is a similar in application and design with the schema detection algorithms. For schema validation the algorithms are trained to predict the target system using post-schema-mapping data elements as input.

According to an embodiment, the reporting instructions 467 direct the system to provide, via the user interface of the system, one or more of the identified source data schema, at least some of the data transformed to the target schema, and any identified mapping anomalies. Other information relevant to the system may also be provided. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands. According to an embodiment, the display may further comprise many other types of information.

According to an embodiment, the data mapping system 400 is configured to process many millions of datapoints in the training of the schema detection algorithm 463, schema mapping algorithm 464, and validation algorithm 466. For example, generating a functional and skilled trained algorithm from a corpus of training data requires processing of millions of datapoints from input data and generated features. This can require millions or billions of calculations to generate a novel trained algorithm from those millions of datapoints and millions or billions of calculations. As a result, each trained algorithm is novel and distinct based on the input data and parameters of the machine learning algorithm, and thus improves the functioning of the data mapping system. And the data mapping system described or otherwise envisioned herein comprises at least three different trained machine learning algorithms and can possibly include numerous others. Generating these functional and skilled fully trained algorithms comprises a process with a volume of calculation and analysis that a human brain cannot accomplish in a lifetime, or multiple lifetimes.

In addition, the data mapping system 400 is configured to process many millions of datapoints in the performance of method 300 in Fig. 3. Obtaining and extracting a corpus of data from one or more data sources, analyzing the normalized data set using the first trained machine learning algorithm to identify the source data schema, deriving mapping rules using a second trained machine learning algorithm, analyzing data transformed to the target schema using a schema validation algorithm to identify any mapping anomalies, and providing information via the user interface require millions or billions of calculations and thus comprises a process with a volume of calculation and analysis that a human brain cannot accomplish in a lifetime, or multiple lifetimes.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A computer-implemented method (300) for mapping data between a source data schema and a target data schema, comprising:
extracting (320) and normalizing data from one or more data sources (470) to generate a normalized data set (462), wherein the one or more data sources comprises the source data schema;
analyzing (330), using a first trained machine learning algorithm (463), the normalized data set to identify the source data schema, wherein identifying the source data schema comprises identifying a general schema level, a data record level, and a data element level for the source data schema;
deriving (340), using a second trained machine learning algorithm (464), mapping rules configured to transform data from the identified source data schema to the target schema, wherein the mapping rules comprises mapping rules at a general schema level, a data record level, and a data element level of the target schema;
transforming (350), using the derived mapping rules, at least some data of the identified source data schema to data of the target schema;
analyzing (360), using a schema validation algorithm (466), the data transformed to the target schema to identify any mapping anomalies; and
providing (370), to a user via a user interface, one or more of the identified source data schema, at least some of the data transformed to the target schema, and any identified mapping anomalies.

2. The method of claim 1, further comprising receiving (380), from the user via the user interface, feedback regarding the mapping rules and/or identified mapping anomalies.

3. The method of claim 2, further comprising modifying (390), based on the received feedback, one or more of the mapping rules.

4. The method of any one of claims 1-3, further comprising updating (390) one or more of: the first trained machine learning algorithm, the second trained machine learning algorithm, and the schema validation algorithm.

5. The method of any one of claims 1-4, wherein at least one of the first trained machine learning algorithm and the second trained machine learning algorithm comprises a deep learning neural network.

6. The method of any one of claims 1-5, wherein one or more of the first trained machine learning algorithm and the second trained machine learning algorithm comprises a natural language processing neural network.

7. The method of any one of claims 1-6, wherein one or more of the first trained machine learning algorithm and the second trained machine learning algorithm comprises a graph neural network.

8. The method of any one of claims 1-7, wherein the one or more data sources comprises both structured and unstructured data which are both normalized to a shared consistent form making up the normalized data set.

9. A system (400) configured to map data between a source data schema and a target data schema, comprising:
a data interface configured to access one or more data sources (470);
the data interface configured to extract a normalized data set (462) from the one or more data sources (470);
a first trained machine learning algorithm (463) trained to analyze the normalized data set to identify the source data schema, wherein identifying the source data schema comprises identifying a general schema level, a data record level, and a data element level for the source data schema;
a second trained machine learning algorithm (464) trained to derive mapping rules configured to transform data from the identified source data schema to the target schema, wherein the mapping rules comprises mapping rules at a general schema level, a data record level, and a data element level of the target schema;
a schema validation algorithm (466) trained to analyze data transformed to the target schema to identify any mapping anomalies;
a processor (420) configured to: (i) analyze, using the first trained machine learning algorithm, the normalized data set to identify the source data schema; (ii) derive, using a second trained machine learning algorithm, mapping rules configured to transform data from the identified source data schema to the target schema; (iii) transform, using the derived mapping rules, at least some data of the identified source data schema to data of the target schema; (iv) analyze, using the schema validation algorithm, the data transformed to the target schema to identify any mapping anomalies; and
a user interface (440) configured to provide one or more of the identified source data schema, at least some of the data transformed to the target schema, and any identified mapping anomalies.

10. The system of claim 9, wherein the user interface is further configured to receive, from a user, feedback regarding the mapping rules and/or identified mapping anomalies.

11. The system of claim 10, wherein process is further configured to modify, based on the received feedback, one or more of the mapping rules.

12. The system of any one of claims 9-11, wherein one or more of the first trained machine learning algorithm and the second trained machine learning algorithm comprises a deep learning neural network.

13. The system of any one of claims 9-12, wherein one or more of the first trained machine learning algorithm and the second trained machine learning algorithm comprises a natural language processing neural network.

14. The system of any one of claims 9-13, wherein one or more of the first trained machine learning algorithm and the second trained machine learning algorithm comprises a graph neural network.

15. A computer program comprising instructions which, when the program is executed by a computer, cause the computer to carry out the steps of the method of any one of claims 1-8.
